# EUROPEAN PATENT SPECIFICATION

(11) **EP 4 210 621 B1**
(45) Date of publication and mention of the grant of the patent: **24.12.2025**
(21) Application number: 21773354.2
(22) Date of filing: 07.09.2021
(51) Int. Cl.: A61B 42/50, A47G 25/90, B65D 83/08

(54) **A GLOVE AND A GLOVE DISPENSING AND DONNING APPARATUS**
HANDSCHUH UND VORRICHTUNG ZUM SPENDEN UND ANZIEHEN VON HANDSCHUHEN
GANT ET APPAREIL DE DISTRIBUTION ET D'ENFILAGE DE GANT

(30) Priority: 09.09.2020 DK PA202001020
(43) Date of publication of application: 19.07.2023
(73) Proprietor: 3L Office Products A/S, 5690 Tommerup (DK)
(72) Inventor: KNUDSEN, Carsten, 9240 Nibe (DK)
(74) Representative: Larsen & Birkeholm A/S
(86) International application number: PCT/EP2021/074541
(87) International publication number: WO 2022/053445

(56) References cited:
- WO-A2-2017/106680
- US-A1- 2005 155 133
- US-A1- 2007 150 996
- US-A1- 2019 387 819

## Description

### Field of the Invention

The invention relates to a system comprising a glove and a glove dispensing and donning apparatus, said system comprises: a disposable glove comprising an opening for hand entry, said glove having an upper opening edge and a lower opening edge, one of them, being provided with a flap, which can be torn off the apparatus without destabilizing or destroying the glove; a glove dispensing and donning apparatus comprising: a first and a second grabber, each having an arm affixed to a base with a mount connected to the arms, said mount comprising moving means adapted such that one or both arms are moveable between a first position in which the grabbers are placed in proximity and a second position in which the grabbers are spaced apart, said glove dispensing and donning apparatus being provided with a holder for at least one disposable glove, said holder fixating the disposable glove such that the first grabber is in contact with the upper opening edge while the second grabber is in functional contact with the lower opening edge of a disposable glove placed in the holder while said arms are in their first position, fastening means are provided on said grabbers such that said grabbers cause the opening for hand entry to expand thereby facilitating the insertion of a hand into said opening for hand entry when said grabbers are moved from their first to their second position

### Background of the Invention

Thin gloves, generally constructed of plastic, have been available commercially for several years. Such gloves are quite inexpensive and are intended to be used by any individual involved in activities where there is a possibility of transfer of disease or infection. Today, disposable gloves are available for free use in many supermarkets. The actual delivery takes place through dispensers from which the customer takes a glove and then applies it to his/her hand.

The typical dispenser consists of a box containing several folded gloves and the costumer/user typically must pull a single glove out through an opening in the box. However, it is quite common to - unintentionally- extract several gloves at a time which commonly results in the unneeded gloves being discarded. As disposable gloves are made of thin plastic, it is often almost impossible for a user to open the glove, to insert his/her hand, without touching the outside of the glove which of course means that pathogenic contaminants can be transmitted from the user to all the objects the glove subsequently comes in contact with. Or worse, users may even be tempted to apply saliva to their fingers to get a better grip, when this happens, contaminants in the form of viruses and bacteria will inevitably be transmitted from the user's mouth to the outside of the glove. After all, it is often a cumbersome and time-consuming procedure to simply put on a pair of gloves on one's hands and therefore users are often tempted to skip this step and simply refrain from using disposable gloves.

From US2019387819 A1 is a disposable glove known. The disposable glove includes a first layer of thermoplastic material and a second layer of thermoplastic material. The first layer of thermoplastic material has a first perimeter border generally in the shape of a human hand and includes a first middle section configured to span interchangeably a palmar aspect and a dorsal aspect of a user's hand based on whether the user's hand is left hand or right hand. The second layer of thermoplastic material has a second perimeter border generally in the shape of a human hand and includes a second middle section configured to span interchangeably a palmar aspect and a dorsal aspect of a user's hand based on whether the user's hand is a left hand or right hand. The first layer and the second layer are coupled together at and along the first perimeter border and the second perimeter border in such a way that results in the disposable glove. The disposable glove has a biased open cuff opening configured to receive a user's left hand or a user's right hand therethrough, wherein the first middle section and the second middle section have different total areas from each other in such a way that one of the first middle section and second middle section contain extra material and does not lay flat, causing the biased open cuff opening to maintain a steady-state open configuration facilitating donning of the disposable glove.

From WO2017106688 A2 is known a glove donning system and a glove packaging assembly. This invention dispenses and holds open a clean glove, allowing a user to don the glove without touching the outside of the glove, thus preventing possible contamination of the outside of the glove. Unlike a traditional glove box, the innovation also keeps other stored gloves that are ready to be dispensed from becoming contaminated, as these gloves remain protected until ready to don.

US2007150996 AA describes right and left hand gloves forming pairs of disposable gloves are adapted to be dispensed from a single dispenser and are further configured to facilitate donning of each of the gloves with one hand so that a pair of gloves can be conveniently donned at one time. With such a configuration, refilling the glove dispensers and donning of a pair of gloves is less cumbersome than known disposable gloves. The right and left hand gloves may be coupled together by various conventional methods to form a glove pair that can be dispensed from a single dispenser. Each glove is formed from two sheets of plastic configured with a thumb portion and at least one finger portion. The bottom sheet includes a residual portion which remains on the dispenser after the glove is removed. The residual portion and the bottom sheet of the glove are separated by a line of weakness, such as a perforation. The residual portions of both the right and left hand gloves may be bound together with a binder, which may include a pair of holes for use with a dispenser. Alternatively, the bottom sheets of the right and left hand gloves may be formed from a continuous sheet of plastic with the mounting holes and lines of weakness disposed therebetween. In order to facilitate donning of each of the gloves with one hand, the glove opening of each glove is spaced away from the line of weakness. By configuring the right and left hand gloves to be dispensed by a single dispenser, both gloves can be essentially refilled at the same time and donned at the same time. In addition, the gloves in accordance with the present invention reduced the cost since only a single dispenser is required.

US2005155133 A1 describes a glove donning method, a system for carrying out the method, and a glove used therewith. More specifically, this invention relates to a method and system for donning a thin glove especially suitable for use in a food processing plant and the like and fabricated from a substantially unstretchable material, such as polyethylene, and relates to such a glove used therewith.

### Object of the Invention

There is a need for a dispenser for disposable gloves where the dispenser facilitates the installation of the glove onto one's hand thereby making the installation of the glove convenient and quick.

The objective of the present invention is to provide a solution that solves at least some of the above problems.

### Summary of the Invention

This is achieved by a system according to the invention as defined by independent claim 1, with further embodiments defined in the dependent claims.

The first grabber comprises means, which hold the upper opening edge and the second grabber holds the flap attached to the lower opening edge of the glove, the second grabber being adapted such that it holds the flap such that it is torn off the apparatus when a hand inserted into the glove is moved away from the apparatus, the first grabber holds the upper edge of the glove in a releasable grip that it is released when a hand inserted into the glove is moved away from the apparatus.

Disposable gloves with flaps are often delivered in a stack and the flaps may be provided with one or more through holes. the holes are used in the prior art to hang the stack on thereto adapted stick (s) or simply in a string (s) that go through the hole (s). When the user wishes to use a glove, the glove with flap is simply torn off the pins or string. If such stacks of gloves are to be used in a system according to the invention, flap gloves can be attached to the apparatus in a similar manner and the apparatus can of course be provided with thereto adapted stick (s) such that a glove with flaps is torn off these stick (s) when a user with his inserted hand pulls sufficiently in the glove

In an embodiment of the invention, the flap and glove are detachably connected such that the flap can be torn of the glove without destabilizing or destroying the glove, the second grabber being adapted such that it holds the flap such that it is torn off the glove along the lower opening edge when a hand inserted into the glove is moved away from the apparatus, the first grabber being adapted such that it holds the upper edge of the glove in a releasable grip that it is released when a hand inserted into the glove is moved away from the apparatus. When many gloves with flaps are stacked in the apparatus it is easy to lock these to the apparatus by simply holding the stack. The detachable connection entails easy release of the glove.

In an embodiment of the invention, the flap and glove are detachably connected along a perforated line. Gloves can be destroyed due to imperfect tears. Perforated lines enable the user to tear "along the dotted line" with ease and without destroying the glove.

In an embodiment of the invention, the apparatus comprises foot pedal operatively connected the first and/or the second grabber (s) such that the first and/or second grabbers can be moved between their first and second positions by means of the foot pedal. By using a foot pedal, any hand contact with the apparatus is avoided, thus reducing the risk of spreading infection. By providing the apparatus with a console, it is further achieved that the operating means (foot pedal) can be placed at a distance from the gloves which also reduces the risk of spreading impurities / bacteria / viruses to the outside of gloves or from one user to another user.

In an embodiment of the invention, the first grabber is operatively connected to a foot pedal such that the first grabber can be moved by means of the foot pedal and cause the opening for hand entry to expand thereby facilitating the insertion of a hand into said opening for hand entry. when only the first grabber is moved by the foot pedal, the number of moving parts is reduced and thus also the risk of malfunction.

In an embodiment of the invention the first grabber is operatively connected to a foot pedal by a rod connection. By connecting the foot pedal to the first grabber via a rod connection, a rigid connection is obtained where the foot pedal can force the grabber in both directions. Thereby a very safe and reliable connection is obtained and the risk of unintentional locking of the first grabber is reduced.

In an embodiment of the invention, the glove is provided with separate sections for each of the fingers and the thumb. Although gloves with fingers are often a little more difficult to put on, they are conversely also very user-friendly as they are similarly easy to hold on the hand. And since the invention solves the problem of applying the glove, the use of the apparatus and finger gloves entails continuous use of gloves.

In an embodiment of the invention, the glove is a glove provided with separate sections for each of the fingers and two thumbs, one of them being placed on the right side of the glove and the other being placed on the left side of the glove, when viewed from the palm of the hand with the fingers pointing either forward or backward. One problem associated with the prior art is that it can be difficult to apply gloves to both the right and left hand with the same device / gloves. This is because in all its simplicity, the thumbs face in opposite directions on the human hands, which is why you must rotate one hand if you want to apply gloves to both hands with prior art apparatus and gloves. A thumb placed on each side of the glove ensures that the user can orient the palms of both hands in the same way and still apply gloves to both hands.

In an embodiment of the invention the holder is provided with fixation means, which holds the glove such that a section for a finger is kept stretched until a user has inserted his or her hand. When gloves made of soft thin plastic lies freely, they will easily move and may curl, which is why they can be difficult to apply to a hand. When a section for a finger is held stretched, the movement of the glove is limited and thus also the possibilities for curling.

In an embodiment of the invention, the holder is provided with fixation means, which holds the glove such that the section for the middle finger is kept stretched until a user has inserted his or her hand. By holding the glove in the lower opening edge with the second grabber and at the same time keeping the middle finger extended, the glove will have an orientation which to some extent resembles the one it has, when it is applied to a hand where the fingers are stretched. The extended middle finger will thus facilitate the insertion of the hand into the glove.

In an embodiment of the invention the second grabber is provided with means adapted for the fixation of a stack of flaps. If the disposable glove/gloves are placed in a stack so that the flaps are placed on top of each other, all the flaps can be held by the second grabber while the gloves are successively applied and torn off one by one from the flaps.

In an embodiment of the invention, the first grabber is provided with a repositionable adhesive, which holds the upper edge of the glove firmly in place and allows the glove to be repositioned without damage to either the glove or the grabber. Many types of glue can hold items in such a way that these can be put on another item several times and taken off again and still retain its sticky properties. These types of adhesives are known to those skilled in the art. A low-tack pressure-sensitive adhesive on the first grabber allow gloves to be easily attached, removed and new gloves to be attached without leaving residue on the gloves.

In an embodiment of the invention the first grabber is provided with a repositionable sticking material, which doesn't make use of adhesives, such materials may be soft, rubber-like materials with very high friction coefficients. It may also be suction or materials provided with small suction cups or the so-called microsuction tape. These solutions are attractive as they do not contain chemical adhesive. This means that the entire apparatus, including grabbers, can be cleaned and after cleaning they can be reused and furthermore, that sterilizing cleaning agents can be used, which contain e.g. alcohol, without destroying the grabber's ability to hold gloves.

In the description and claims is the word "donning" used. Donning means in this application to put on the gloves or the process of applying the gloves. "A donning apparatus" is an apparatus which assist in the process of applying or putting on gloves.

In the description and claims a glove is described. The word glove describe in this application gloves with separate sections for one or more fingers or without any separate sections for one or more fingers.

### BRIEF DESCRIPTION OF THE DRAWINGS

In the following the invention will be described in greater detail with reference to exemplary embodiments in accordance with the accompanying drawings, in which:
fig. 1 shows in perspective an embodiment of the invention;
fig. 2 shows, in perspective and in greater details, the grabber shown in figure 1;
fig. 3 shows in perspective the invention comprising a foot pedal;
fig. 4 shows in perspective an embodiment of the invention comprising a finger holder;
fig. 5 shows in perspective the invention comprising a finger holder;
fig. 6 shows in perspective the invention comprising a finger holder.

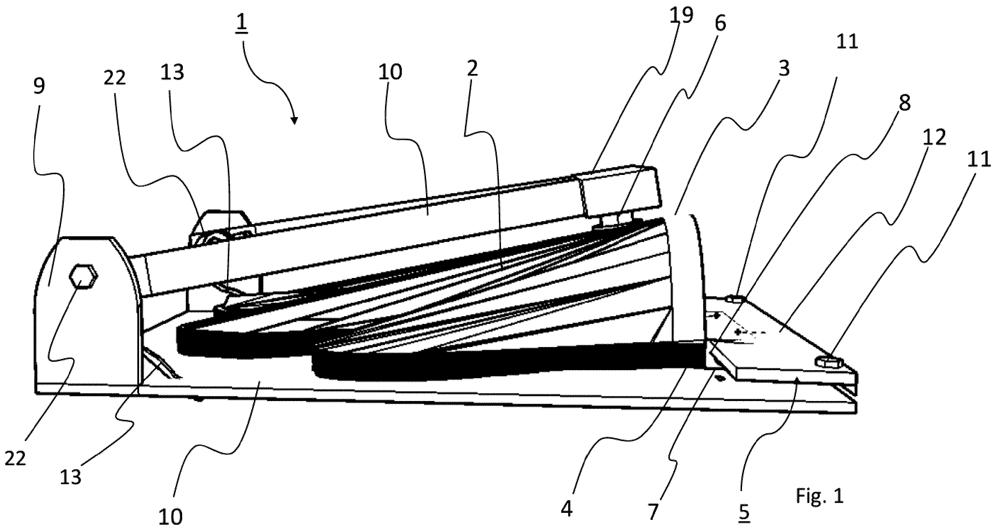

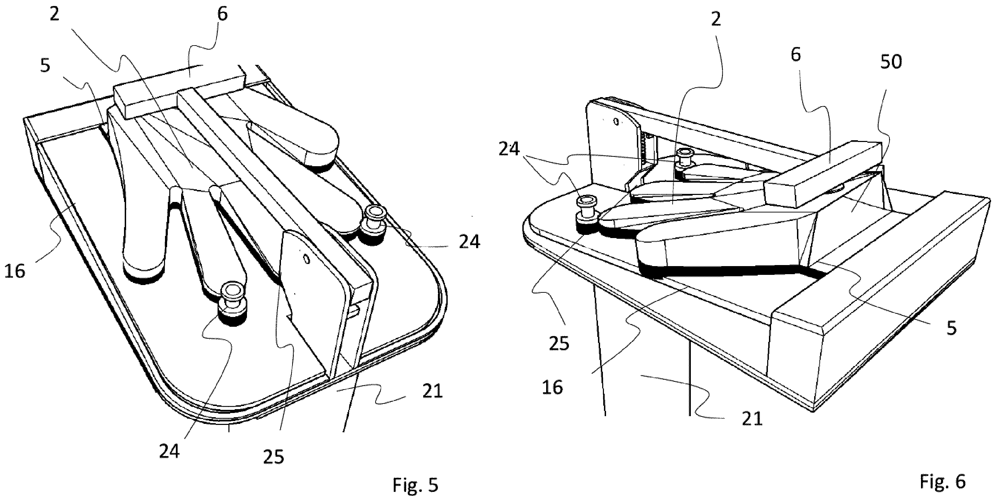

In figure 1 is shown a glove and a glove dispensing and donning apparatus 1 comprising a disposable glove 2 provided with an opening for hand entry. The opening is not shown explicitly in figure 1, however, a glove 2 is shown in the figure with the hand entry 50 opened (the hand entry is shown in figure 6). The glove's wrist part defines an upper opening edge 3 and a lower opening edge 4. One of the edges is provided with a flap 7. In the shown embodiment is the lower opening edge 4 provided with the flap 7, which can be torn off (the apparatus or the glove) without destabilizing or destroying the glove. The glove shown in figure 1 is provided with a flap 7, of which only a very small part can be seen in figure 1.

The glove dispensing and donning apparatus comprising: a first 6 and a second grabber 5, each having an arm 10 affixed to a base 9 comprising a mount 22 connected to the arms. The mount 22 comprising moving means adapted such that one or both arms can be moved between a first position in which the grabbers 5, 6 are placed in proximity and a second position in which the grabbers are spaced apart. In the shown embodiment the mount 22 comprises two bolts 22 which allow the arms to rotate in relation to each other such that the grabbers 5, 6 can be brought into and out of close relationship. The glove dispensing and donning apparatus is provided with a holder (not shown in embodiment shown in figure 1) for at least one disposable glove 2. The disposable glove 2 is placed such that the first grabber is in contact with the gloves upper opening edge 3, while the second grabber is in functional contact with the glove's lower opening edge. The disposable glove shown in figure 1 has its hand entry open and the arms are shown in their second position. Each grabber is provided with fastening means connected to the upper edges 3 and lower edges 4 such that the glove is maintained in the open position until a user inserts his or her hand into the opening in order to put on the glove and subsequently moves the gloved hand away.

Gloves with flaps as shown in figure 1 are often used in supermarkets and they are delivered in stacks. The flaps serve the purpose of holding the stack of gloves and when a user needs a glove, it is torn off the flap. Therefore, gloves and flaps are often assembled at the glove opening by a perforation 8 line that makes tearing easier. The flaps are normally provided only on one side of the gloves and this fact is utilized by the invention in that the lower (second) grabber 2 of the apparatus holds the entire stack of flaps. The stack of flaps in the shown embodiment is fastened by the first grabber 6 which comprises a top plate 12. The stack of flaps is secured between the top plate 12 and the lower arm 10 when the top plate 12 is forced down towards the right side of the lower arm 10 by means of the bolt-connection 11.

The second grabber is placed on the holder such that the second grabber holds the flap attached to the lower opening edge of the glove. The flap is torn off the glove along the lower opening edge when a hand inserted into the glove is moved away from the holder.

The first grabber comprises means, which hold the upper opening edge in such a way that it releases the glove when a hand inserted into the glove is moved away from the holder. The means for the fixation of the first grabber to the upper edge of the glove may in an embodiments of the invention be a repositionable adhesive, however other means may be used to hold the upper edge of the glove while the glove is opened and a hand is inserted into the glove.

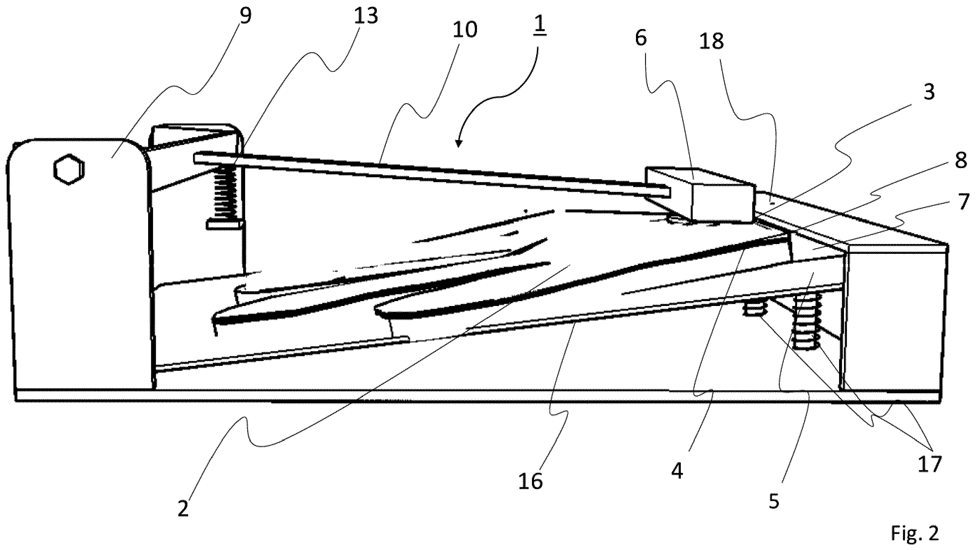

In figure 2 is shown another embodiment of the invention. The arm holding the second grabber is in this embodiment a plate 16. The gloves 2 comprising flaps 7 are- in the shown embodiment - retained in the apparatus by the flaps being clamped between the plate 16 and a top plate 18. The clamping force is achieved by springs 17, which press the plate 16 against the top plate 18. The first grabber 6 and its associated arm connection 9, 10 is provided with a second spring connection 13, which is arranged in the base 9 of the apparatus. This spring connection 13 is adapted in that it acts on the arm 10 of the first grabber in such a way that the first and second grabber will be located in the position where they are separated when they aren't affected by any other forces than gravity, even when a glove is "sticking" to the grabber.

This technical arrangement has the advantage that the user simply must lower the first grabber 6 so that it touches the top glove in the stack and then releases it.

Hereby the glove will first attach to the grabber (due to the adhesive / fastening means on this) and then open. In use this means that the user simply has to lower the first grabber, let go of the grabber and then stick his/her hand into the glove 2.

The user then tears the glove from the flap / stack by simply moving his hand away in such a way that the hand is not pulled out of the glove. E.g. vertically up, with clasped hand or further in the insertion direction etc.

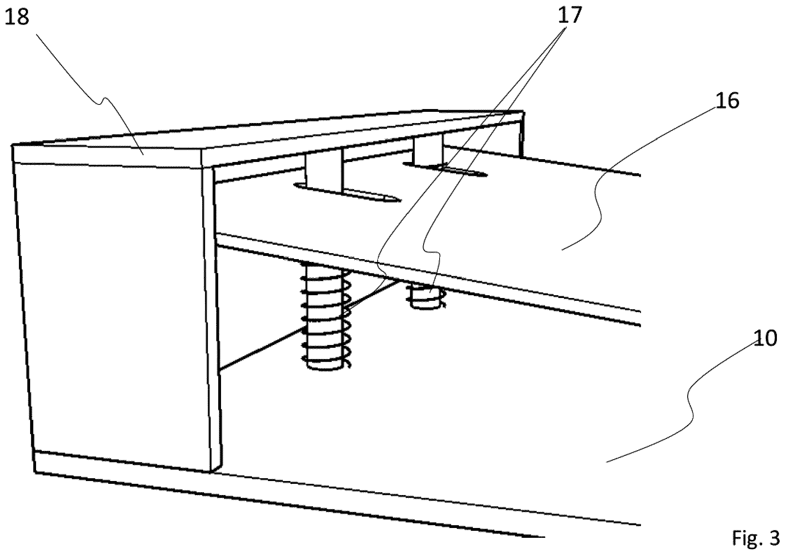

Figure 3 shows in greater details how the clamping arrangement with springs 17, the plate 16 and the top plate shown in figure 2 can be made.

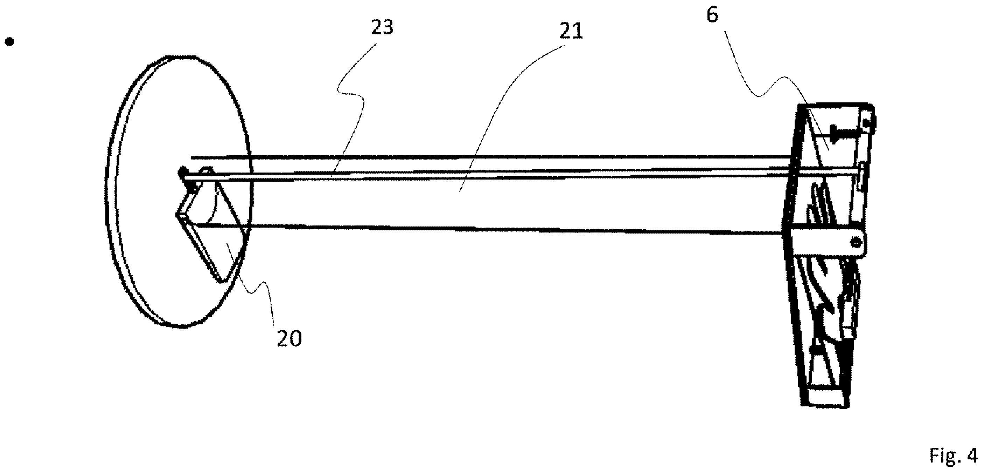

Figure 4 shows an embodiment wherein the first grabber is made movable by a foot pedal 20. In this embodiment, the invention comprises a foot pedal 20, a stand, a column, and a rod connection. In this embodiment a food pedal 20 is provided and this pedal 20 is via a rod connection 23 connected to the first grabber such that the first grabber can be moved by use of the foot pedal. This connection comprises a column 21 and a rod 23.

Figures 5 and 6 show embodiments where the holder for gloves 2 is provided with fixation means 24, which holds the glove such that the section is kept in the same position until a user has inserted his or her hand.

In the embodiments shown in figure 5 and 6 the glove's 2 middle finger 25 is fixated. By holding the glove in the upper and lower opening edges with the first 6 and second grabber and at the same time keeping the middle finger extended, the glove will have an orientation, which to some extent resembles the one it has when it is applied to a hand where the fingers are stretched. The fixated extended middle finger will thus facilitate the insertion of the hand into the glove. If the plate 16 is inclined as shown in the figure 5 and 6 gravity will also affect the gloves placed in the device and thereby entails an orientation, which to some extent resembles the one it has when it is applied to a hand where the fingers are stretched.

## Claims

1. A system comprising a glove and a glove dispensing and donning apparatus (1), said system comprises:
• a disposable glove (2) comprising an opening (50) for hand entry, said glove (2) having an upper opening edge (3) and a lower opening edge (4), one of them, being provided with a flap (7), which can be torn off the apparatus without destabilizing or destroying the glove (2);
• a glove (2) dispensing and donning apparatus (1) comprising: a first (6) and a second grabber (5), each having an arm (10) affixed to a base (9) with a mount (22) connected to the arms,(10) said mount (22) comprising moving means adapted such that one or both arms (10) are moveable between a first position in which the grabbers (5, 6) are placed in proximity and a second position in which the grabbers are spaced apart, said glove (2) dispensing and donning apparatus being provided with a holder for at least one disposable glove, said holder fixating the disposable glove (2) placed in the holder while said arms (10) are in their first position, fastening means are provided on said grabbers such that said grabbers cause the opening for hand entry to expand thereby facilitating the insertion of a hand into said opening for hand entry when said grabbers are moved from their first to their second position,
**characterized in that,**
said holder fixating the disposable glove (2) such that the first grabber is in contact with the upper opening edge (3) while the second grabber is in functional contact with the lower opening edge (4) of a disposable glove (2), the first grabber comprises means, which holds the upper opening edge (3) and the second grabber holds the flap attached to the lower opening edge (4) of the glove (2), the second grabber being adapted such that it holds the flap such that it is torn off the apparatus when a hand inserted into the glove (2) is moved away from the apparatus, the first grabber being adapted such that it holds the upper edge (3) of the glove (2) in a releasable grip that it is released when a hand inserted into the glove (2) is moved away from the apparatus.

2. A system according to claim 1, wherein, the flap and glove (2) are detachably connected such that the flap can be torn of the glove (2) without destabilizing or destroying the glove (2), the second grabber being adapted such that it holds the flap such that it is torn off the glove (2) along the lower opening edge (4) when a hand inserted into the glove (2) is moved away from the apparatus, the first grabber being adapted such that it holds the upper edge (3) of the glove (2) in a releasable grip that it is released when a hand inserted into the glove (2) is moved away from the apparatus.

3. A system according to claim 1 or 2, wherein, the flap (7) and glove (2) are detachably connected along a perforated line.

4. A system according to any of the claims 1 -3 , wherein the glove (2) is a glove (2) provided with separate sections for each of the fingers and the thumb.

5. A system according to any of the claims 1-4, wherein the glove (2) is a glove (2) provided with separate sections for each of the fingers and two thumbs, one of them being placed on the right side of the glove (2) and the other being placed on the left side of the glove (2), when viewed from the palm/over side of the glove (2) with the fingers pointing either forward or backward.

6. A system according to any of the claims 1-5, wherein the glove (2) dispensing and donning apparatus is provided with a foot pedal (20) operatively connected to the first and/or the second grabber (s) such that the first and/or second grabbers can be moved between their first and second positions by means of the foot pedal (20)

7. A system according to any of the claims 1-6, wherein the glove (2) dispensing and donning apparatus is provided with a foot pedal, operatively connected to the first grabber such that the first grabber can be moved between first and second position by means of the foot pedal.

8. A system according to claims 6 or 7, wherein the first grabber (6) is operatively connected to a foot pedal (20) by a rod connection (23).

9. A system according to any of the claims 1-8, wherein the holder is provided with fixation means (24), which hold the glove (2) such that a section for a finger is kept stretched until a user has inserted his or her hand.

10. A system according to any of the claims 1-9, wherein the holder is provided with fixation means (24), which hold the glove (2) such that the section for the middle finger (25) is kept stretched until a user has inserted his or her hand.

11. A system according to any of the claims 1-10, wherein the second grabber is provided with means adapted for the fixation of a stack of flaps.

12. A system according to any of the claims 1-11, wherein the first grabber is provided with a repositionable adhesive, which holds the upper edge of the glove (2) firmly in place and allows the glove (2) to be repositioned without damage to either the glove (2) or the grabber.

13. A system according to any of the claims 1-12, wherein the first grabber is provided with a repositionable holding material, which does not make use of adhesives.

## Patentansprüche

1. System, umfassend einen Handschuh und eine Vorrichtung (1) zum Spenden und Anziehen von Handschuhen, wobei das System Folgendes umfasst:
einen Einweghandschuh (2), umfassend eine Öffnung (50) zum Einführen der Hand, wobei der Handschuh (2) eine obere Öffnungskante (3) und eine untere Öffnungskante (4) aufweist, von denen eine mit einer Lasche (7) bereitgestellt ist, die von der Vorrichtung abgerissen werden kann, ohne den Handschuh (2) zu destabilisieren oder zu zerstören;
eine Vorrichtung (1) zum Spenden und Anziehen von Handschuhen (2), umfassend: einen ersten (6) und einen zweiten Greifer (5), die jeweils einen Arm (10) aufweisen, der an einer Basis (9) befestigt ist, mit einer Halterung (22), die mit den Armen (10) verbunden ist, die Halterung (22) umfassend Bewegungsmittel, die ausgelegt sind, derart, dass einer oder beide Arme (10) zwischen einer ersten Position, in der die Greifer (5, 6) nahe beieinander angeordnet sind und einer zweiten Position, in der die Greifer voneinander beabstandet sind, beweglich sind, wobei die Vorrichtung zum Spenden und Anziehen von Handschuhen (2) mit einem Halter für mindestens einen Einweghandschuh bereitgestellt ist, wobei der Halter den in den Halter eingesetzten Einweghandschuh (2) fixiert, während sich die Arme (10) in ihrer ersten Position befinden, wobei Befestigungsmittel an den Greifern bereitgestellt sind, derart, dass die Greifer veranlassen, dass sich die Öffnung zum Einführen der Hand erweitert, wodurch das Hineinführen einer Hand in die Öffnung zum Einführen der Hand erleichtert wird, wenn die Greifer von ihrer ersten zu ihrer zweiten Position bewegt werden,
**dadurch gekennzeichnet, dass**,
der Halter den Einweghandschuh (2) fixiert, derart, dass der erste Greifer mit der oberen Öffnungskante (3) in Kontakt steht, während der zweite Greifer in funktionellem Kontakt mit der unteren Öffnungskante (4) eines Einweghandschuhs (2) steht,
der erste Greifer Mittel umfasst, die die obere Öffnungskante (3) halten und der zweite Greifer die an der unteren Öffnungskante (4) des Handschuhs (2) befestigte Lasche hält, wobei der zweite Greifer ausgelegt ist, derart, dass er die Lasche hält, derart, dass sie von der Vorrichtung abgerissen wird, wenn eine in den Handschuh (2) eingeführte Hand von der Vorrichtung wegbewegt wird, wobei der erste Greifer ausgelegt ist, derart, dass er die obere Kante (3) des Handschuhs (2) in einem lösbaren Griff hält, der freigesetzt wird, wenn eine in den Handschuh (2) eingeführte Hand von der Vorrichtung wegbewegt wird.

2. System nach Anspruch 1, wobei die Lasche und der Handschuh (2) lösbar verbunden sind, derart, dass die Lasche vom Handschuh (2) abgerissen werden kann, ohne den Handschuh (2) zu destabilisieren oder zu zerstören, wobei der zweite Greifer ausgelegt ist, derart, dass er die Lasche hält, derart, dass sie entlang der unteren Öffnungskante (4) vom Handschuh (2) abgerissen wird, wenn eine in den Handschuh (2) eingeführte Hand von der Vorrichtung wegbewegt wird, wobei der erste Greifer ausgelegt ist, derart, dass er die obere Kante (3) des Handschuhs (2) in einem lösbaren Griff hält, der freigesetzt wird, wenn eine in den Handschuh (2) eingeführte Hand von der Vorrichtung wegbewegt wird.

3. System nach Anspruch 1 oder 2, wobei die Lasche (7) und der Handschuh (2) entlang einer Perforationslinie lösbar miteinander verbunden sind.

4. System nach einem der Ansprüche 1 bis 3, wobei der Handschuh (2) ein Handschuh (2) ist, der mit separaten Abschnitten für jeden der Finger und den Daumen bereitgestellt ist.

5. System nach einem der Ansprüche 1 bis 4, wobei der Handschuh (2) ein Handschuh (2) ist, der mit separaten Abschnitten für jeden der Finger und zwei Daumen bereitgestellt ist, von denen einer auf der rechten Seite des Handschuhs (2) und der andere auf der linken Seite des Handschuhs (2) angeordnet ist, wenn man von der Handfläche/Oberseite des Handschuhs (2) aus betrachtet, wobei die Finger entweder nach vorne oder nach hinten zeigen.

6. System nach einem der Ansprüche 1 bis 5, wobei die Vorrichtung zum Spenden und Anziehen von Handschuhen (2) mit einem Fußpedal (20) bereitgestellt ist, das funktionsfähig mit dem ersten und/oder dem zweiten Greifer verbunden ist, derart, dass der erste und/oder der zweite Greifer durch das Fußpedal (20) zwischen ihrer ersten und zweiten Position bewegt werden können.

7. System nach einem der Ansprüche 1 bis 6, wobei die Vorrichtung zum Spenden und Anziehen von Handschuhen (2) mit einem Fußpedal bereitgestellt ist, das funktionsfähig mit dem ersten Greifer verbunden ist, derart, dass der erste Greifer durch das Fußpedal zwischen einer ersten und einer zweiten Position bewegt werden kann.

8. System nach Anspruch 6 oder 7, wobei der erste Greifer (6) über eine Stabverbindung (23) mit einem Fußpedal (20) verbunden ist und betrieben werden kann.

9. System nach einem der Ansprüche 1 bis 8, wobei der Halter mit Befestigungsmitteln (24) bereitgestellt ist, die den Handschuh (2) halten, derart, dass ein Abschnitt für einen Finger gestreckt gehalten wird, bis ein Benutzer seine oder ihre Hand eingeführt hat.

10. System nach einem der Ansprüche 1 bis 9, wobei der Halter mit Befestigungsmitteln (24) bereitgestellt ist, die den Handschuh (2) halten, derart, dass der Abschnitt für den Mittelfinger (25) gestreckt gehalten wird, bis ein Benutzer seine oder ihre Hand eingeführt hat.

11. System nach einem der Ansprüche 1 bis 10, wobei der zweite Greifer mit Mitteln bereitgestellt ist, die zum Fixieren eines Stapels von Laschen ausgelegt sind.

12. System nach einem der Ansprüche 1 bis 11, wobei der erste Greifer mit einem repositionierbaren Klebstoff bereitgestellt ist, der die Oberkante des Handschuhs (2) fest an seinem Platz hält und es ermöglicht, den Handschuh (2) ohne Beschädigung des Handschuhs (2) oder des Greifers neu zu positionieren.

13. System nach einem der Ansprüche 1 bis 12, wobei der erste Greifer mit einem repositionierbaren Haltematerial, das keine Klebstoffe verwendet, bereitgestellt ist.

## Revendications

1. Système comprenant un gant et un appareil de distribution et d'enfilage (1) de gant, ledit système comprend :
• un gant jetable (2) comprenant une ouverture (50) pour l'introduction d'une main, ledit gant (2) ayant un bord supérieur d'ouverture (3) et un bord inférieur d'ouverture (4), l'un d'eux étant muni d'un rabat (7), qui peut être détaché de l'appareil sans déstabiliser ou détruire le gant (2) ;
• appareil de distribution et d'enfilage (1) de gant (2) comprenant : un premier (6) et un second dispositif de préhension (5), chacun ayant un bras (10) fixé à une base (9) par un support (22) relié aux bras (10), ledit support (22) comprenant un moyen de déplacement adapté de sorte que l'un ou les deux bras (10) soient mobiles entre une première position, dans laquelle les dispositifs de préhension (5, 6) sont rapprochés, et une seconde position, dans laquelle les dispositifs de préhension sont espacés, ledit appareil de distribution et d'enfilage de gant (2) étant muni d'un dispositif de maintien pour au moins un gant jetable, ledit dispositif de maintien fixant le gant jetable (2) placé dans le dispositif de maintien lorsque lesdits bras (10) sont dans leur première position, des moyens de fixation sont prévus sur lesdits dispositifs de préhension de sorte que lesdits dispositifs de préhension provoquent l'élargissement de l'ouverture pour l'introduction d'une main, facilitant ainsi l'insertion d'une main dans ladite ouverture d'introduction de main lorsque lesdits dispositifs de préhension sont déplacés de leur première à leur seconde position,
**caractérisé en ce que** :
ledit dispositif de maintien fixe le gant jetable (2) de sorte que le premier dispositif de préhension soit en contact avec le bord supérieur d'ouverture (3) tandis que le second dispositif de préhension est en contact fonctionnel avec le bord inférieur d'ouverture (4) d'un gant jetable (2),
le premier dispositif de préhension comprend un moyen qui maintient le bord supérieur d'ouverture (3) et le second dispositif de préhension maintient le rabat attaché au bord inférieur d'ouverture (4) du gant (2), le second dispositif de préhension étant adapté de manière à maintenir le rabat de sorte qu'il soit détaché de l'appareil lorsqu'une main insérée dans le gant (2) est éloignée de l'appareil, le premier dispositif de préhension étant adapté de manière à maintenir le bord supérieur (3) du gant (2) dans une prise libérable de sorte qu'il soit libéré lorsqu'une main insérée dans le gant (2) est éloignée de l'appareil.

2. Système selon la revendication 1, dans lequel le rabat et le gant (2) sont reliés de manière détachable de sorte que le rabat puisse être détaché du gant (2) sans déstabiliser ou détruire le gant (2), le second dispositif de préhension étant adapté de manière à maintenir le rabat de sorte qu'il soit détaché du gant (2) le long du bord inférieur d'ouverture (4) lorsqu'une main insérée dans le gant (2) est éloignée de l'appareil, le premier dispositif de préhension étant adapté de manière à maintenir le bord supérieur (3) du gant (2) dans une prise libérable de sorte qu'il soit libéré lorsqu'une main insérée dans le gant (2) est éloignée de l'appareil.

3. Système selon la revendication 1 ou 2, dans lequel le rabat (7) et le gant (2) sont reliés de manière détachable le long d'une ligne perforée.

4. Système selon l'une quelconque des revendications 1 à 3, dans lequel le gant (2) est un gant (2) muni de sections séparées pour chacun des doigts et le pouce.

5. Système selon l'une quelconque des revendications 1 à 4, dans lequel le gant (2) est un gant (2) muni de sections séparées pour chacun des doigts et des deux pouces, l'un d'entre eux étant placé sur le côté droit du gant (2) et l'autre étant placé sur le côté gauche du gant (2), vu depuis la paume/le dessus du gant (2) avec les doigts pointant vers l'avant ou vers l'arrière.

6. Système selon l'une quelconque des revendications 1 à 5, dans lequel l'appareil de distribution et d'enfilage de gant (2) est muni d'une pédale (20) reliée de manière opérationnelle au premier et/ou au second dispositif(s) de préhension de sorte que les premier et/ou second dispositifs de préhension puissent être déplacés entre leurs première et seconde positions au moyen de la pédale (20).

7. Système selon l'une quelconque des revendications 1 à 6, dans lequel l'appareil de distribution et d'enfilage de gant (2) est muni d'une pédale, reliée de manière opérationnelle au premier dispositif de préhension de sorte que le premier dispositif de préhension puisse être déplacé entre la première et la seconde position au moyen de la pédale.

8. Système selon les revendications 6 ou 7, dans lequel le premier dispositif de préhension (6) est relié de manière opérationnelle à une pédale (20) par une liaison à tige (23).

9. Système selon l'une quelconque des revendications 1 à 8, dans lequel le dispositif de maintien est muni de moyens de fixation (24) qui maintiennent le gant (2) de sorte qu'une section pour un doigt soit maintenue tendue jusqu'à ce qu'un utilisateur ait inséré sa main.

10. Système selon l'une quelconque des revendications 1 à 9, dans lequel le dispositif de maintien est muni de moyens de fixation (24) qui maintiennent le gant (2) de sorte que la section pour le majeur (25) soit maintenue tendue jusqu'à ce qu'un utilisateur ait inséré sa main.

11. Système selon l'une quelconque des revendications 1 à 10, dans lequel le second dispositif de préhension est muni de moyens adaptés à la fixation d'une pile de rabats.

12. Système selon l'une quelconque des revendications 1 à 11, dans lequel le premier dispositif de préhension est muni d'un adhésif repositionnable, qui maintient fermement le bord supérieur du gant (2) en place et permet au gant (2) d'être repositionné sans endommager ni le gant (2) ni le dispositif de préhension.

13. Système selon l'une quelconque des revendications 1 à 12, dans lequel le premier dispositif de préhension est muni d'un matériau de maintien repositionnable, qui n'utilise pas d'adhésifs.
